(19) Europäisches Patentamt
European Patent Office
Office européen des brevets

(11) EP 1 709 954 A1

(12) EUROPEAN PATENT APPLICATION

(43) Date of publication:
**11.10.2006 Bulletin 2006/41**

(21) Application number: **05007389.9**

(22) Date of filing: **05.04.2005**

(51) Int Cl.:
***A61K 8/29*** *(2006.01)* ***A61K 8/27*** *(2006.01)*
***A61K 8/892*** *(2006.01)* ***A61Q 1/02*** *(2006.01)*
***A61Q 17/04*** *(2006.01)* ***A61K 8/28*** *(2006.01)*

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(71) Applicant: **THE PROCTER & GAMBLE COMPANY**
**Cincinnati, Ohio 45202 (US)**

(72) Inventors:
- **Elliott, Russell Phillip**
  **Virginia Water**
  **Surrey GU25 4EU (GB)**
- **Hodgetts, Jennifer Clare**
  **Addlestone**
  **Surrey KT15 2JX (GB)**

(74) Representative: **Wilding, Richard Alan et al**
**Procter & Gamble Technical Centres Limited**
**Patent Department**
**Rusham Park**
**Whitehall Lane**
**Egham, Surrey TW20 9NW (GB)**

(54) **Translucent, sunscreening cosmetic foundation composition**

(57) A cosmetic foundation composition is provided having an SPF value of greater than or equal to 15, preferably greater than or equal to 18, more preferably greater than or equal to 20 and a $\Delta L_T$ value greater than or equal to 0.5, preferably greater than or equal to 1.0, more preferably greater than or equal to 2.5, where $\Delta L_T$ is measured according to the following equation:

$$\Delta L_T = \Delta L_2 - \Delta L_1$$

Where:

$$\Delta L_1 = L_1^{110} - L_1^{15};$$

$$\Delta L_2 = L_2^{110} - L_2^{15}$$

$L_1$ is a measurement of $L^y$ taken on bare skin, to which no product has been applied;
$L_2$ is a measurement of $L^y$ taken on skin, to which product has been applied; and
$L^y$ is the translucency value at a specific angle, y, from specular.

EP 1 709 954 A1

**Description**

FIELD OF THE INVENTION

The present application concerns translucent cosmetic foundation compositions which bestow a high sunscreening benefit.

BACKGROUND OF THE INVENTION

Cosmetic skin foundations and cosmetic sunscreen products are known and have been for many years. It has also been known for some time to produce cosmetic skin foundations which provide a sunscreening benefit. Formulators of such foundations are, however, faced with certain apparently contradictory challenges - to increase the ability of the foundation to obscure the underlying skin and to provide a satisfactory sunscreening benefit on the one hand, while, on the other hand, not bestowing an extremely artificial look on the skin. Preserving the natural appearance of the skin, however, is often best achieved by employing a relatively translucent product which allows the underlying skin to be seen — the apparent opposite of obscuring it. Where metal oxide sunscreens are being relied upon to provide the sunscreening benefit, reducing the amounts of such materials to give a translucent product may also diminish the UV blocking ability of that product.

The problems are compounded by the fact that, with age, the translucency of skin diminishes, such that maturer users of cosmetic skin foundations often express the desire for their foundations actually to increase apparent translucency. Currently, the only approach which addresses the issue of translucency of skin is to reduce the coverage, i.e. to reduce the concentration of metal oxide pigment in the composition, such that on application, less skin is actually obscured. At best this approach can approximately maintain the inherent translucency of the skin beneath, but, as can be readily understood, such foundations are unable to provide sufficient coverage of the target surface and may not be able to provide sufficient sunscreening benefit either. A small additional increase in apparent translucency may sometimes be achievable by adding a shiny emollient to the composition, but such increases are insufficient to meet the needs of the consumer.

The present invention addresses the above shortcomings in the prior art by providing a foundation that provides both a high coverage of the underlying skin and a high sunscreening benefit while, at the same time, retaining and even enhancing translucency to maintain a natural appearance.

SUMMARY OF THE INVENTION

According to the invention, a cosmetic foundation composition is provided having an SPF value of greater than or equal to 15, preferably greater than or equal to 18, more preferably greater than or equal to 20 and a $\Delta L_T$ value greater than or equal to 0.5, preferably greater than or equal to 1.0, more preferably greater than or equal to 2.5, where $\Delta L_T$ is measured according to the following equation:

$$\Delta L_T = \Delta L_2 - \Delta L_1$$

Where:

$$\Delta L_1 = L_1^{110} - L_1^{15};$$

$$\Delta L_2 = L_2^{110} - L_2^{15}$$

$L_1$ is a measurement of $L^y$ taken on bare skin, to which no product has been applied;
$L_2$ is a measurement of $L^y$ taken on skin, to which product has been applied; and
$L^y$ is the translucency value at a specific angle, y, from specular.

BRIEF DESCRIPTION OF THE DRAWINGS

While the specification concludes with claims which particularly point out and distinctly claim the invention, it is believed the present invention will be better understood from the following description of preferred embodiments taken in conjunction with the accompanying drawings, in which like reference numerals identify identical elements and in which:

Fig. 1 is an illustration of a hiding power chart, discussed in greater detail below in relation to the method of calculating $\Delta L$.

Fig. 2 is a diagram illustrating how light incident at 45° to a skin surface reflects off that surface. Again, this diagram is discussed in greater detail below in relation to the method of calculating $\Delta L$.

DETAILED DESCRIPTION OF THE INVENTION

All weights, measurements and concentrations herein are measured at 25°C on the composition in its entirety, unless otherwise specified.

Unless otherwise indicated, all percentages of compositions referred to herein are weight percentages of the total composition (i.e. the sum of all components present) and all ratios are weight ratios.

As used herein in relation to metal oxide particles, all weights of doping or coating materials are given as percentages of the weight of the underlying metal oxide particle which is thus doped or coated. This definition applies even when the doping or coating material is, itself, a metal oxide. Thus, if the particles weigh x grammes and the coating or doping material weighs y grammes, the percentage weight of the coating or doping material is y/x * 100.

As used herein in relation to the cosmetic foundation composition, the percentage weight of the metal oxide sunscreen particles is the combined weight of the underlying metal oxide particle and any doping or coating divided by the weight of the entire cosmetic foundation composition. Thus, if the particles weigh x grammes, the coating or doping material weighs y grammes and the entire cosmetic foundation composition (including the coated or doped metal oxide particles) weighs z grammes, then the percentage weight of the metal oxide particle is (x + y)/z * 100.

Unless otherwise indicated, all polymer molecular weights are number average molecular weights.

Reference herein to the percentage weight of cross-linked organopolysiloxane elastomer in a composition is a reference to the percentage weight of solid organopolysiloxane elastomer in that composition.

Unless otherwise indicated, the content of all literature sources referred to within this text are incorporated herein in full by reference.

Except where specific examples of actual measured values are presented, numerical values referred to herein should be considered to be qualified by the word "about".

Translucency may be regarded as a measure of the way a surface upon which light is shone handles that light. To be more specific, it may be regarded as the difference in light intensity that an observer would see by observing the same part of a surface from different angles, when the surface is illuminated at a constant angle, such as 45° from the normal. Empirically, when under constant illumination at 45°, the most extreme differences are observed when the same part of the surface is observed from 15° (often called the "chroma band") and 110° (backscattered light) angles, measured from the specular reflectance band of the 45° incident light (not measured from the normal in this case). This is illustrated in Figure 2, discussed in greater detail hereinbelow. As a result, the difference in the light intensity measured at 15° and 110° may be regarded as a measure of translucence, with high positive values being indicative of high translucence and low positive or even negative values being indicative of low translucence surfaces.

Skin, especially young, healthy skin, has a relatively high translucence which means that there is a large difference between light intensity measured at 15° and 110° (more light is absorbed at 15° than 110°). This effect also may be explained by the composition of the skin, which allows light to enter and leave the skin resulting in different light path lengths according to the measurement angle - this follows from the Beer-Lambert Law.

Turning back to the issue in hand (the perception by consumers of skin coated with foundation), it is evident that covering skin with metal oxide particles has the effect of increasing light scattering in all directions, including at 15° and 110°, by preventing light from entering the skin. Since the path length of the light scattered at 15° is decreased more than that scattered at 110° (in fact, at 110°, there is little decrease in path length) the increase in light intensity at 15° is dramatically larger than at 110° such that the perceived translucence decreases with increasing coverage with metal oxide. A challenge faced by the present inventors was to retain or even increase the skin coverage by metal oxide particles while at the same time increasing perceived translucence.

Following translucency measurements of currently marketed cosmetic foundation compositions, it has been established that none of those tested can achieve translucence, $\Delta L_T$, of greater than or equal to 0.5 in combination with the high sunscreening benefit achieved by the invention. Preferably, the $\Delta L_T$ value is greater than or equal to 1.0, more preferably greater than or equal to 2.5, more preferably still greater than or equal to 4.0.

Advantageously, cosmetic foundation compositions according to the invention do not have a translucence, $\Delta L_T$, value greater than 30.

One way of increasing the translucency of skin covered with foundation is to include cross-linked organopolysiloxane elastomer in the composition. Without wishing to be bound by theory, such elastomers may create islands of reduced opacity in the dried-down film through which light may be transmitted to the skin. Although cross-linked organopolysiloxane elastomers are preferred for use herein, other materials which achieve the same effect may be employed in their stead.

Cross-linked organopolysiloxane elastomer may be present in cosmetic foundation compositions according to the invention in an amount from 0.01% to 15%, preferably from 1% to 10%, more preferably from 2 to 5% by weight of the cosmetic foundation composition.

No specific restriction exists as to the type of curable organopolysiloxane composition that can serve as starting material for the cross-linked organopolysiloxane elastomer. Examples in this respect are addition reaction-curing organopolysiloxane compositions which cure under platinum metal catalysis by the addition reaction between SiH-containing diorganopolysiloxane and organopolysiloxane having silicon-bonded vinyl groups; condensation-curing organopolysiloxane compositions which cure in the presence of an organotin compound by a dehydrogenation reaction between hydroxyl-terminated diorganopolysiloxane and SiH-containing diorganopolysiloxane; condensation-curing organopolysiloxane compositions which cure in the presence of an organotin compound or a titanate ester, by a condensation reaction between an hydroxyl-terminated diorganopolysiloxane and a hydrolyzable organosilane (this condensation reaction is exemplified by dehydration, alcohol-liberating, oxime-liberating, amine-liberating, amide-liberating, carboxyl-liberating, and ketone-liberating reactions); peroxide-curing organopolysiloxane compositions which thermally cure in the presence of an organoperoxide catalyst; and organopolysiloxane compositions which are cured by high-energy radiation, such as by gamma-rays, ultraviolet radiation, or electron beams.

Preferred organopolysiloxane elastomers are dimethicone/vinyl dimethicone crosspolymers. Such dimethicone/vinyl dimethicone crosspolymers are supplied by a variety of suppliers including Dow Coming (DC 9040, DC 9040 and DC 9045), General Electric (SFE 839 and the Velvesil range of products), Shin Etsu (KSG-15, 16, 18, [dimethicone/phenyl vinyl dimethicone crosspolymer]), and Grant Industries (Gransil(TM) line of materials), lauryl dimethicone/vinyl dimethicone crosspolymers supplied by Shin Etsu (e.g. KSG-320, KSG-41, KSG-42, KSG-43, and KSG-44) and dimethicone/PEG-10 crosspolymer, such as KSG-210 and 240. Highly preferred organopolysiloxane elastomers are DC9040, KSG15, KSG-210 and KSG320.

Cosmetic foundation compositions according to the invention may additionally comprise an dispersant, which has the function of wetting the metal oxide pigment particles to prevent agglomeration. With reference to the translucency model discussed above, agglomeration can be considered to increase the translucency at 15° (because there is an increase in random scattering of light from the surface), thereby decreasing the difference in light intensity between 15° and 110°, which in turn decreases the perceived translucence. Reducing or avoiding agglomeration counters this effect. Dispersants which may be employed according to the invention may be selected from the group consisting of classes A, B, and C, as defined below, or mixtures of these materials:

Class A materials are:

(a) are non-volatile; and
(b) have a viscosity less than or equal to $5cm^2/s$ (500 centistokes), preferably less than or equal to $1cm^2/s$ (100 centistokes), more preferably less than or equal to 0.5 $cm^2/s$ (50 centistokes) at 25°C; and
(c) have a dielectric constant from 3.0 to 5.0, preferably from 3.5 to 5.0.

Examples of dispersants belonging to Class A include branched esters of diglycerin or triglycerin or the esters or 1,2,3,4 butane triol or erythritol, di erythritol or tri erthyritol. These esters must have at least one free hydroxyl group. Preferred dispersants in this class include erythrityl triethylhexanoate (available as Salacos E-38 from Nisshin Oilio) and Polyglyceryl-2 triisostearate (available as Cosmol 43V from Nisshin Oilio).

As used herein, the term "non-volatile" when employed in relation to dispersants includes materials that fulfil at least one of the following definitions: (a) the oil exhibits a vapour pressure of no more than about 0.2 mm Hg at 25°C and one atmosphere pressure; (b) the oil has a boiling point at one atmosphere of at least about 300°C.

Viscosity is measured on a Brookfield RV++ spindle at speed 100rpm and 25°C.

The dielectric constant of the dispersant was measured at 20°C using a Model 870 liquid dielectric constant meter manufactured by Scientifica in Princeton NJ. Readings were taken once equilibrium had been reached (as a rule, it took five minutes to achieve a constant value).

Class B materials adhere to the formula R - X - R', wherein R and R' are $C_6$-$C_{10}$ alkyl groups and X is an oxygen atom or a carbonate group, i.e. a group having the structure:

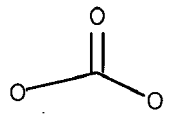

Preferably, X is a carbonate group.
R may be identical to or different from R'.
Preferably, R and R' are, independently, straight or branched chain $C_7$-$C_9$.
More preferably, both R and R' are $C_8$ alkyl groups.
More preferably still, both R and R' are 2-ethylhexyl groups and X is a carbonate group.
Commercially available dispersants falling within this class include TEGOSOFT DEC (Goldschmidt AG), CETIOL OE (Cognis AG) and CETIOL CC.

Class C comprises only isononyl isononanoate (available as Lanol 99 from Seppic).

In addition to wetting the metal oxide pigment particles to reduce or prevent agglomeration, dispersants may additionally have the effect of providing a reflective surface layer over the metal oxide particles. Without wishing to be bound by theory, it is believed that these materials may ensure that the applied cosmetic foundation composition has a microscopically smooth surface.
Cosmetic foundation compositions according to the invention may comprise from 0.1%wt to 20%wt, preferably from 0.5%wt to 10%wt, more preferably from 1%wt to 8%wt , more preferably still from 2%wt to 6%wt dispersant.
Cosmetic compositions according to the invention comprise metal oxide particles. Any suitable metal oxide may be employed. Preferably, the metal oxide particles are selected from the group consisting of titanium oxide, zinc oxide, zirconium oxide, yellow iron oxide, black iron oxide, red iron oxide, chromium oxide, chromium hydroxide, zirconium oxide and cerium oxide.
Cosmetic foundation compositions according to the invention may comprise from 0.1 wt% to 50 wt%, preferably from 0.1 to 30wt%, more preferably from 1 to 20wt% metal oxide particles, which quantity comprises metal oxide pigments and also, if present, metal oxide sunscreen particles.
Metal oxide pigments that may be employed include Anatase pigments, such as X200 from Kemira Oy (Finland) and Rutile pigments, such as CR50 or PF 671 from ISK Japan. X200 is available as a 65% methicone treated dispersion from Kobo Products Inc as FA65UMLO.
Minute metal oxide particles have a highly reactive surface that can cause unwarranted chemical or photochemical reactions. To counter this effect, it is known to dope these surfaces with one or more other materials such as silica, or metal oxides, such as alumina, to reduce the reactivity of the surface. This surface treatment may typically represent from 15 to 30% by weight of the metal oxide particle. Advantageously metal oxide particles comprised within cosmetic compositions according to the invention may be so-doped.
Advantageously, the metal oxide particles may be provided with a hydrophobic coating to improve the particles' dispersion in hydrophobic carrier medium. Advantageously, the metal oxide particles comprise from 2 to 25%, preferably from 5% to 15%, more preferably from 7% to 12% hydrophobic coating by weight of the metal oxide particles.
Advantageously, the hydrophobic coating may be made by applying a mixture of one or more of the following materials and isopropyl alcohol onto the metal oxide powder and drying at 150°C for 3 hours: reactive organo-polysiloxane, polyolefin (including polyethylene and polypropylene), hydrogenated lecithin and salts thereof, N-acylamino acid and salts thereof and dextrin fatty acid esters. Preferably, the reactive organo-polysiloxane comprises organo hydrogen polysiloxane, triorgano siloxy silicic acid and organopolysiloxane modified at both terminal ends with trialkoxy groups. Commercially available materials falling into the category of reactive organo-polysiloxanes include KF-99, KF-9901, KF-7312F, KF-7312-J, KF-7312K, KF-9001, KF-9002, X-21-5249 and X-21-5250 manufactured by the Shin-Etsu Chemical Company Ltd; SH-1107, DC593, BY-11-015, BY-11-018 and BY-11-022 manufactured by Dow Coming Toray Silicone Co. Ltd.; TSF484, TSF483 and TSF4600 manufactured by Toshiba Silicone Co. Ltd.; FZ3704 and AZ6200 manufactured by Nippon Unicar Co. Ltd.
The hydrophobic coating is not limited to those described in the preceding paragraph and alternative hydrophobic coatings known to the skilled person may be employed instead. Such coatings may include trialkoyl isopropyl titanate,

preferably triisostearoyl isopropyl titanate and perfluoro coatings, preferably polyperfluoroethoxymethoxy PEG-2 phosphate.

As has already been discussed, the invention may make use of islands of reduced opacity, which comprise organopolysiloxane elastomer. By definition, these "islands" allow not only visible light in and out to give the desired translucency benefit, but also UV light. Allowing UV light to reach the skin may militate against a high sunscreening benefit, however, thereby reducing the SPF value achievable by the product. In order to counteract this effect, defined SPF-enhancing mechanisms may be employed as discussed below. These SPF-enhancing mechanisms give rise to compositions having an SPF value of greater than or equal to 15, preferably greater than or equal to 18, more preferably greater than or equal to 20. SPF is a commonly used measure of photoprotection of a sunscreen against erythema and is defined as a ratio of the ultraviolet energy required to produce minimal erythema on protected skin to that required to products the same minimal erythema on unprotected skin in the same individual (see Federal Register, Vol. 64, No 98, pp. 27666 - 27693, 21 May, 1999). According to the invention, each SPF-enhancing mechanism may be used on its own, or it may be used in combination with another SPF-enhancing mechanism.

According to the first SPF-enhancing mechanism, specific measures are taken to increase the sunscreening properties of the film around the "islands" of reduced opacity. Although, on the face of it, such a mechanism might not be considered to have a significant effect on the amounts of UV-light reaching the skin, surprisingly the opposite is the case. Without wishing to be bound by theory, it is believed that the "islands" of elastomer scatter a significant proportion of the UV-light incident upon them into the surrounding film, so that, if that light can be effectively blocked there, it will be prevented from reaching the skin.

According to a first embodiment of the first SPF-enhancing mechanism, cosmetic foundations according to the invention comprise first metal oxide sunscreen particles having a first number weighted average primary particle size from 1nm to 50nm and second metal oxide sunscreen particles having a second number weighted average primary particle size which is greater than 50nm and less than or equal to 200nm. By having both the defined smaller and larger metal oxide sunscreen particles present, three objectives may be achieved simultaneously - a UVB sunscreening effect (achieved by the larger particles), a UVA sunscreening effect (achieved by the smaller particles) and improved skin coverage due to increased reflection of visible light (also by the larger particles). The larger particles may therefore act simultaneously as sunscreens and as pigments.

The first and second metal oxide sunscreen particles may comprise any suitable metal oxides which give a sunscreening effect and each of the first and second metal oxides may be oxides of a single metal or mixtures of two or more metals (provided that the number weighted average primary particle size conditions are adhered to). Preferably, the first and second metal oxide particles are selected from the group consisting of titanium oxide particles, zinc oxide particles and mixtures thereof. More preferably still, the first and second metal oxide particles comprise titanium dioxide particles.

Advantageously, the first number weighted average primary particle sizes range from 5 to 40nm and the second number weighted average primary particle sizes range from greater than 50nm to 150nm. More advantageously, the first number weighted average primary particle size is from 8 to 30nm and the second number weighted average secondary particle size is from greater than 50nm to 120nm, preferably from greater than or equal to 55nm to 100nm.

The first metal oxide particles advantageously comprise from 0.1 to 10%, preferably from 0.5 to 8%, more preferably from 1 to 5% of the composition and the second metal oxide particles advantageously comprise from 0.1 to 10%, preferably from 0.5 to 8%, more preferably from 2 to 5% of the composition.

Advantageously, compositions according to the present invention comprise a higher percentage weight of the second metal oxide particles than of first metal oxide particles such that the weight ratio:

$$\frac{\text{weight of first metal oxide particles}}{\text{weight of second metal oxide particles}}$$

is less than 1.0 and is in the range 0.99 to 0.1. This may be especially advantageous in the case of cosmetic compositions which are intended to mask the underlying skin to some degree, such as cosmetic skin foundations: by having more of the larger particles present, three objectives may be achieved simultaneously - a UVB sunscreening effect, a UVA sunscreening effect and improved skin coverage due to increased reflection of visible light by the higher proportion of larger particles.

Commercially available materials which may be employed as first metal oxide particles include M262 from Kemira Oy. and TTO S-3, TTO S-4 , TTO V-3 from Ishihara Corp. Commercially available materials which may be employed as second metal oxide particles include KQ-1, MPT-140 and MPT141 from Ishihara Corp. In a preferred embodiment, SAS-TTO S-3/D5 from Miyoshi Kasei (which comprises fibril-coated TTO S-3 particles), which have an average primary particle of about 15nm, and SAS-KQ-1 (which comprises fibril-coated KQ-1 particles), with a primary particle size of about 60nm, may be combined as first and second metal oxide particles respectively.

As used herein, the term "primary particle size" means metal oxide crystal size, as determined by x-ray diffraction.

It is based on measuring the broadening of the strongest rutile line.

According to a second embodiment of the first SPF-enhancing mechanism, hydrophilic (water soluble) organic and/or water dispersible organic sunscreens are included in the composition. By definition, these materials will migrate away from the lipophilic silicone elastomer "islands" into other parts of the film, where, in use, they tend to crystallise out on "dry-down" (the period during which the water in the product evaporates following application to the skin). To this end, any hydrophilic or water dispersible organic sunscreen may be employed. Non-limiting examples of preferred hydrophilic organic sunscreens include PBSA (2-Phenylbenzimidazole-5-sulfonic acid), Benzophenone-4 (2-Benzoyl-5-Methoxy-1-Phenol-4-Sulphonic Acid) and PABA (Para Amino Benzoic Acid). A non-limiting example of a preferred water dispersible organic sunscreen is methylene bis-benzotriazolyl tetramethylbutylphenol (Available in a 50% dispersion as Tinosorb M from Ciba). Cosmetic foundation compositions according to the invention may comprise comprise 0.1-8%, preferably 0.5-6%, more preferably 1% to 5% hydrophilic organic sunscreen, water-dispersible organic sunscreen or mixtures thereof.

According to the second SPF-enhancing mechanism, lipophilic organic sunscreen is included in the product. Without wishing to be limited by theory, it is believed that the lipophilic organic sunscreen may become solubilized within the "islands" of organopolysiloxane elastomer, thereby directly blocking UV-light from reaching the skin through those regions of reduced opacity. To this end, any lipophilic organic sunscreen may be employed - suitable examples may be found in the CTFA International Cosmetic Ingredient Dictionary and Handbook, 7th edition, volume 2 pp. 1672, edited by Wenninger and McEwen (The Cosmetic, Toiletry, and Fragrance Association, Inc., Washington, D. C., 1997). It is preferred, however, to use non-polar lipophilic organic sunscreens such as 2-ethylhexyl-2-cyano-3, 3-diphenylacrylate (referred to as octocrylene), cinnamates and their derivatives such as 2-ethylhexyl-p-methoxycinnamate and octyl-p-methoxycinnamate and homosalate, octylsalicylate, octyldiemthyl para amino benzoic acid and diethylamino hydroxybenzoylhexyl benzoate (Uvinul A Plus).

Without wishing to be bound by theory, it is believed that polar organic sunscreens may interact with the pigment particles to cause agglomeration, whereas the non-polar materials listed do not give rise to such difficulties. Cosmetic foundation compositions according to the invention may comprise from 1 to 20%, preferably from greater than 2% to less than or equal to 15%, more preferably from 3% to 15% lilophilic organic sunscreen by weight of the cosmetic foundation composition. Where the lipophilic organic sunscreen is octyl methoxycinnamate, it is preferred that it be present in an amount that is greater than 2% by weight of the cosmetic foundation composition.

Advantageously, cosmetic foundation compositions according to the invention comprise an oil. Oil may be present in an amount from 1% to 80% by weight of the cosmetic foundation composition.

The oil may be selected from the group consisting of silicones, which may be cyclic or linear, functionalised or non-functionalised; organic oils including $C_{10}$ to $C_{30}$ branched, linear or cyclic alkanes or esters and mixtures thereof, but excluding isononyl isononanoate.

Preferred cyclic silicones which may be employed in cosmetic foundation compositions according to the invention correspond to the formula:

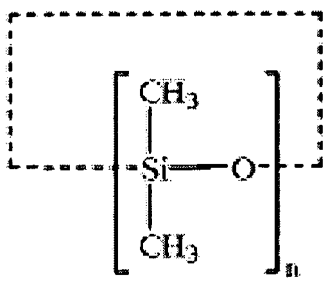

wherein n is from about 3 to about 8. Highly preferably, the cyclic volatile silicones are selected from cyclopentasiloxane, cyclohexasiloxane and mixtures thereof.

Preferred linear silicones which may be employed in cosmetic foundation compositions according to the invention correspond to the formula:

$$(CH_3)_3Si\text{-}O\text{-}[Si(CH_3)_2\text{-}O]_m\text{-}Si(CH_3)_3$$

wherein m is from about 1 to about 20 preferably from 3 to 12.

Linear silicones generally have a viscosity of less than about 50 centistokes, preferably less than 5 centistokes at 25°C; cyclic silicones generally have viscosities of less than about 10 centistokes at 25°C.

Examples of commercially available cyclic silicones include the following: Dow Coming 200, Dow Coming 244, Dow Coming 245, Dow Coming 344, and Dow Coming 345 (commercially available from Dow Coming Corp.); SF-1204 and SF-1202 Silicone Fluids (commercially available from G. E. Silicones), GE 7207 and 7158 (commercially available from General Electric Co.); and SWS-03314 (commercially available from SWS Silicones Corp.).

Preferred examples of linear dimethicones useful include DC200 5cst, DC1630 and DC 5-2117, More preferably; the linear dimethicone comprises DC 5-2117.

Preferred organic oils which may be included in cosmetic foundation compositions according to the invention are isohexadecane, isododecane and mixtures thereof.

Cosmetic foundation compositions according to the invention may be formulated as anhydrous products or as emulsions. If the cosmetic foundation compositions are formulated as emulsions, those emulsions may be water-in-oil (water-in-silicone) emulsions or oil-in-water (silicone-in-water) emulsions, but are preferably water-in silicone emulsions.

Advantageously, the cosmetic foundation compositions according to the invention are formulated as water-in-silicone emulsions that contain from 0.1 to 70%, preferably from 1 to 50%, more preferably from 5 to 40% water.

Cosmetic foundation compositions according to the invention, whether or not they are in the form of an emulsion, may comprise emulsifier. The emulsifier may be selected from the group consisting of nonionic, anionic, cationic, zwitterionic and amphoteric emulsifiers and mixtures thereof. Suitable emulsifiers are disclosed in McCutcheon's Detergents and Emulsifiers, North American Edition, pages 317-324.

In the event that the cosmetic foundation composition according to the invention is a water-in-silicone emulsion, then preferred emulsifiers are selected from the group consisting of polyoxyalkylene copolymers (also known as silicone polyethers), polyglyceryl copolymers and mixtures thereof. Polyoxyalkylene copolymers are described in detail in US 4,268,499. More preferred polyethers include PEG/PPG-18/18 Dimethicone available as blend with cyclopentasiloxane as DC5225C or DC5185; PEG 9 Dimethicone, available as KF6017 or KF6028 from Shin-Etsu. A preferred polyglyceryl emulsifier is available as KF6100 and KF6104 from Shin-Etsu Inc.

In one embodiment, it is preferred that cosmetic foundation compositions according to the invention comprise only polyglyceryl copolymer emulsifiers and no polyoxyalkylene emulsifiers. This is because polyoxyalkylene emulsifiers may break down to release ethylene glycol and aldehydes which may give rise to increased sensitivity on the skin of some consumers.

The total concentration of the emulsifier may be from 0.01% to about 15%, more preferably from about 0.1 % to about 10% of the formulation, even more preferably from 1.0% to about 5% and more preferably still from about 1.0% to about 3%, by weight of the composition.

Cosmetic foundation compositions according to the present invention may optionally contain spherical particles having an average particle diameter from 1 to 50 $\mu$m, preferably from 5 to 20 $\mu$m. As used herein in relation to the spherical particles, the particle diameter shall be understood to be that of primary particles.

Preferred spherical particles include, but are not limited, to polymeric particles chosen from the methylsilsesquioxane resin microspheres such as for example those sold by GE silicone under the name Tospearl 145A or Tospearl 2000; microspheres of polymethylmethacrylates such as those sold by Seppic under the name Micropearl M 100; the spherical particles of crosslinked polydimethylsiloxanes, especially such as those sold by Dow Coming Toray Silicone under the name Trefil E 506C or Trefil E 505C, spherical particles of polyamide and more specifically Nylon 12, especially such as those sold by Atochem under the name Orgasol 2002D Nat C05, polystyerene microspheres such as for example those sold by Dyno Particles under the name Dynospheres, ethylene acrylate copolymer sold by Kobo under the name FloBead EA209 and mixtures thereof. Also found to be useful is Ronasphere LDP from Kobo Inc. Polyurethane particles BPD500 sold by Kobo Inc. may also be employed.

If present, the spherical particles may be included in the cosmetic foundation compositions according to the invention at a concentration of from about 0.01% to about 40%, more preferably from about 1% to about 10%, more preferably still from about 1%to about 5%.

Cosmetic foundation compositions according to the present invention may further comprise a skin-conditioning agent. These agents may be selected from humectants, exfoliants or emollients and may be present from about 0.01% to 30%, preferably from about 1% to about 20%, more preferably from about 1% to 10% by weight of the cosmetic foundation composition.

Humectants which may be included in cosmetic foundation compositions according to the invention include polyhydric alcohols such as glycerine, propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerine and mixtures thereof. Most preferably the humectant comprises glycerine.

In addition, hydrophilic gelling agents such as those selected from the group consisting of the acrylic acid/ethyl acrylate copolymers, carboxyvinyl polymers (such as those sold by the B.F. Goodrich Company under the Carbopol trademark, polyacrylamides (such as those available from Seppic as Seppigel 305) and mixtures thereof may be included

in the cosmetic foundation compositions according to the invention.

A variety of additional optional ingredients may be incorporated into the compositions of the present invention. Non-limiting examples of these additional ingredients include additional skin care actives such as peptides (e.g., Matrixyl [pentapetide derivative]), farnesol, bisabolol, phytantriol, urea, guanidine (e.g., amino guanidine); vitamins and derivatives thereof such ascorbic acid, vitamin A (e.g., retinoid derivatives such as retinyl palmitate or retinyl proprionate), vitamin E (e.g., tocopherol acetate), vitamin $B_3$ (e.g., niacinamide) and vitamin $B_5$ (e.g., panthenol) and the like and mixtures thereof; anti-acne medicaments (resorcinol, salicylic acid, and the like; antioxidants (e.g., phytosterols, lipoic acid); flavonoids (e.g., isoflavones, phytoestrogens); skin soothing and healing agents such as aloe vera extract, allantoin and the like; chelators and sequestrants; and agents suitable for aesthetic purposes such as essential oils, fragrances, skin sensates, opacifiers, aromatic compounds (e.g., clove oil, menthol, camphor, eucalyptus oil, and eugenol).

Method of calculating translucency

All translucency measurements made in the below-defined methods are carried out using an X-Rite™ MA68 II Spectrophotometer. For avoidance of doubt, however, any suitable multi-angle spectrometer could be used, provided it is properly calibrated with the same light source, the same incident light angle and the same reflectance angles (15° and 110° from specular, as defined in Figure 2) are used.

Foundation Standardisation

Colour at 45° incident light is measured on all products using the X-Rite™ MA68 II Spectrophotometer using a CMC (Colour Measurement Committee) tolerancing method which is considered most closely matched to the visual acceptability response of the human eye. Using the CMC equation (see British standard BS:6923) tolerance ellipsoids are calculated around the target in colour space allowing for the assessment of small colour differences between samples. The light source is a $D_{65}$ illuminant at a 10° standard observer. This light source is intended to represent average daylight and has a correlated colour temperature of approximately 6500 K (recognised as a standard by ISO 10526:1999).

With reference to Figure 1, which illustrates a hiding power chart (chart ref: 301/2A supplied by Sheen Instruments Ltd.), product is applied to Region (1) at a thickness of 300 μm and readings are taken over the middle of the hiding power chart in Region (2), where the black and white halves join, and are taken in the direction of the arrow shown on the right hand side of the figure.

All products to be tested must be comparable to shade 'Buff Beige' - shade #111 within the CoverGirl™ foundation palette - (L* = 68.72 a* = 11.47 b* = 19.49). L*, a* and b* refer to co-ordinates in 3D colour space, where L* indicates how light to dark the colour is, a* indicates the position on the red to green axis and b* indicates the position on the yellow to blue axis - these quantities are known to the skilled person in this area and need not be further defined.

The delta E values are calculated vs. this standard using the following equation:

$$\text{Delta E} = \sqrt{[(L - L^*)^2 + (a - a^*)^2 + (b - b^*)^2]}$$

For inclusion into testing, products must be no greater than a delta E of 0.5 vs. the standard. For avoidance of doubt, however, it is not necessary to use CoverGirl™ foundation in order to reproduce this method — any foundation achieving the Delta E value would be acceptable.

Translucency Measurement Method for a Given Product

Reflectance and Delta L are measured on the whole composition *in vivo* on human skin using the X-Rite™ MA68 II, 5-angle spectrophotometer, an industry standard device for analysing reflected light and colour. With reference to Figure 2, the spectrophotometer incident light (3) is at 45° to the skin surface (4), and it analyses reflected light at 15°, 25°, 45°, 75° and 110° away from the specular reflectance (5) (also at 45° to the surface and at 90° to the incident light).

As used herein, $L^y$ is the L-value measured at a specific angle, y, from specular.

As used herein, $L_1$, also referred to as the baseline measurement, is a measurement taken on bare skin, to which no product has been applied

As used herein, $L_2$, also referred to as post-application measurement, is a measurement taken on skin, to which product has been applied.

Given the inherent variability of in vivo data, a base size of at least 15 test persons is required to provide

statistically accurate data. Using multifactor analysis, it is possible to generate data showing highly significant differences between products using the present method. Prior to use, the instrument is calibrated using the white and black standards supplied with the machine. The test persons are Caucasian females aged 18-35 years.

The skin is illuminated with incident light at 45° from normal. The light source is a $D_{65}$ illuminant at a 10° standard observer (as described above). The skin is read three times without product to provide an arithmetical average base-line measurement ($L_1$). The base-line measurement of translucency ($\Delta L_1$) is defined as:

$$\Delta L_1 = L_1^{110} - L_1^{15}$$

The product is then applied to bare skin without markings (such as tattoos) on the forearm at a dosage of 15µl across a 3 cm x 3 cm square area (9 $cm^2$). The product is applied in an even film using a sweeping flat finger motion across a total of 15 swipes. After every 3 sweeps the direction of product wiping is rotated by 90°. The product is then allowed to dry on the skin for 5 minutes. An arithmetical average of three measurements ($L_2$) is then taken for each test person. The post-application measurement of translucency ($\Delta L_2$) is defined as:

$$\Delta L_2 = L_2^{110} - L_2^{15}$$

The base-line measurement, $\Delta L_1$, is then subtracted from the post-application measurement, $\Delta L_2$, to give the change in translucency ($\Delta L_T$)

$$\Delta L_T = \Delta L_2 - \Delta L_1$$

Translucent products have positive values of $\Delta L_T$. Highly translucent products according to the invention have $\Delta L_T$ values greater than or equal to 3.5, preferably greater than or equal to 4.5, more preferably greater than or equal to 5.0. As will be appreciated, for highly matte materials, this number may be negative, as these materials will actually reduce the amount of reflected light.

Examples

The following examples further describe and demonstrate the preferred embodiments within the scope of the present invention. The examples are given solely for the purpose of illustration, and are not to be construed as limitations of the present invention since many variations thereof are possible without departing from its scope.

A liquid foundation of the present invention is prepared as follows: in a suitable vessel, water, glycerine, disodium EDTA and benzyl alcohol are added and mixed using conventional technology until a clear water phase is achieved. When the water phase is clear, the methylparabens are added and mixed again until clear. The resultant phase is mixed with a Silverson SL2T or similar equipment on high speed (8,000 rpm, standard head). In a separate vessel, the KSG21, DC245, Pigment dispersion, other oils, dispersant and the parabens are added and the mixture is milled using a Silverson SL2T on a high speed setting until a homogeneous mixture is created.

Following this step, the water phase and the silicone phase are combined and milled using the Silverson SL2T on a high speed setting until the water is fully incorporated and an emulsion is formed. The remaining elastomer is then added and the mixture is mixed again using the Silverson on a high speed setting to generate the final product.

| **Example #** | **1** | **2** | **3** | **4** |
|---|---|---|---|---|
| Ingredient | | | | |
| DC9040 cross linked elastomer gel (1) | 25.0 | 25.0 | 25.0 | 25.0 |

(continued)

| Example # | 1 | 2 | 3 | 4 |
|---|---|---|---|---|
| KSG 320 cross linked elastomer gel (2) | | | | |
| Dimethicone copolyol cross-polymer (KSG21) (2) | 0.5 | 0.5 | 0.5 | 0.5 |
| Decamethylcyclopentasiloxane (DC245) (1) | 6.0 | 8.5 | 6.0 | 8.15 |
| PEG/PPG18/18 Dimethicone & Cyclomethicone (DC5185) (1) | 2.0 | 2.0 | 2.0 | 2.0 |
| Diethylhexyl carbonate (Tegosoft DEC) (3) | 3.0 | 3.0 | 3.0 | 4.0 |
| Octyl Methoxy cinnamate | 3.0 | 3.0 | 4.0 | |
| Benzophenone 3 | 0.5 | 0.5 | | |
| Benzophenone 4 | 0.5 | 0.5 | | |
| Phenyl benzimidazole Sulfonic Acid methylene bis-benzotriazolyl tetramethylbutylphenol (Tinosorb M 50% Dispersion) | 3.00 | 3.00 | | 3.00 |
| Triethanolamine | 2.00 | 2.00 | | 2.00 |
| Fibril coated sunscreen grade Titanium dioxide 50% dispersion in D5 SAS/TT0-S-3/D5 (4) | 6.0 | | 6.0 | 6.0 |
| Fibril coated sunscreen grade Zinc oxide 60% dispersion in D5 SAMT-UFZO-450 (4) | | 4.00 | | |
| Pigmentary Titanium dioxide (9729) Coated with 2% methicone (5) | | | | |
| Fibril coated UVA sunscreen grade Titanium dioxide 60% dispersion in D5 SAS/KQ-1 /D5 (4) | | 5.00 | 5.00 | 5.00 |
| Pigmentary grade Titanium dioxide 65% dispersion in D5 FA65UMLO | | 8.00 | 8.00 | 8.00 |
| Black Iron Oxide coated with 2% methicone (5)* | 0.22 | 0.22 | 0.22 | 0.22 |
| Yellow Iron Oxide coated with 2% methicone (5)* | 1.2 | 1.2 | 1.2 | 1.2 |
| Red Iron Oxide coated with 2% methicone (5)* | 0.4 | 0.4 | 0.4 | 0.4 |
| Propylparabens | 0.1 | 0.1 | 0.1 | 0.1 |
| Ethylparabens | 0.1 | 0.1 | 0.1 | 0.1 |
| Methylparabens | 0.1 | 0.1 | 0.1 | 0.1 |
| Disodium EDTA | 0.1 | 0.1 | 0.1 | 0.1 |
| Benzyl alcohol | 0.25 | 0.25 | 0.25 | 0.25 |
| Sodium chloride | 2.00 | 2.00 | 2.00 | 2.00 |
| Glycerin | 7.00 | 7.00 | 7.00 | 7.00 |
| Water | qs | qs | qs | qs |
| | | | | |
| SPF | 17 | 17 | 15 | 16 |
| Translucency, $\Delta L_T$ | 4.7 | 4.3 | 4.9 | 5.2 |

(1) Available from Dow Corning
(2) Available from Shin-Etsu
(3) Available from Degussa
(4) Available from Miyoshi Kasei
(5) Available from Sensient
* these are added as slurries in cyclopentasiloxane (D5)

## Claims

1. A cosmetic foundation composition having an SPF-value of greater than or equal to 15, preferably greater than or equal to 18, more preferably greater than or equal to 20 and a $\Delta L_T$ value greater than or equal to 0.5, preferably greater than or equal to 1.0, more preferably greater than or equal to 2.5, where $\Delta L_T$ is measured according to the following equation:

$$\Delta L_T = \Delta L_2 - \Delta L_1$$

Where:

$$\Delta L_1 = L_1^{110} - L_1^{15};$$

$$\Delta L_2 = L_2^{110} - L_2^{15}$$

$L_1$ is a measurement of $L^y$ taken on bare skin, to which no product has been applied;
$L_2$ is a measurement of $L^y$ taken on skin, to which product has been applied; and
$L^y$ is the translucency value at a specific angle, y, from specular.

2. The cosmetic foundation composition of claim 1, comprising from 0.01% to 15%, preferably from 1% to 10%, more preferably from 2% to 5% by weight of the cosmetic foundation composition of cross-linked organopolysiloxane elastomer.

3. The cosmetic foundation composition of claim 2, wherein the cross-linked organopolysiloxane elastomer comprises dimethicone/vinyl dimethicone crosspolymer.

4. The cosmetic foundation composition of any one of the preceding claims additionally comprising a dispersant.

5. The cosmetic foundation of claim 4, wherein the dispersant:

   (a) is non-volatile; and
   (b) has a viscosity less than or equal to 5cm $^2$/s (500 centistokes), preferably less than or equal to 1cm$^2$/s (100 centistokes), more preferably less than or equal to 0.5 cm$^2$/s (50 centistokes) at 25°C; and
   (c) has a dielectric constant from 3.0 to 5.0, preferably from 3.5 to 5.0.

6. The cosmetic foundation of claim 5, wherein the dispersant adheres to the formula R - X - R', wherein R and R' are $C_6$-$C_{10}$ alkyl groups and X is a carbonate group.

7. The cosmetic foundation of claim 6, wherein both R and R' are 2-ethylhexyl groups.

8. The cosmetic foundation of claim 5, wherein the dispersant is isononyl isononanoate.

9. The cosmetic foundation of any one of claims 4 to 8, comprising from 0.1 %wt to 20%wt, preferably from 0.5%wt to 10%wt, more preferably from 1%wt to 8%wt , more preferably still from 2%wt to 6%wt dispersant.

10. The cosmetic foundation composition of any one of the preceding claims additionally comprising metal oxide particles.

11. The cosmetic foundation composition of claim 10, wherein the metal oxide particles are selected from the group consisting of titanium oxide, zinc oxide, zirconium oxide, zirconium oxide, iron oxide and cerium oxide.

12. The cosmetic foundation composition of claim 10 or 11, comprising from 0.1 wt% to 50 wt% metal oxide particles

**13.** The cosmetic foundation composition of any one of claims 10 to 12, comprising first metal oxide sunscreen particles having a first number weighted average primary particle size from 1nm to 50nm, preferably from 5nm to 40nm and second metal oxide sunscreen particles having a second number weighted average primary particle size which is greater than 50nm and less than or equal to 200nm, preferably greater than 50nm and less than or equal to 150nm.

**14.** The cosmetic foundation composition of any one of claims 10 to 13, additionally comprising a water soluble and/or a water dispersible sunscreen.

**15.** The cosmetic foundation composition of claim 14, wherein the water soluble organic sunscreen is selected from the group consisting of 2-Phenylbenzimidazole-5-sulfonic acid, 2-Benzoyl-5-Methoxy-1-Phenol-4-Sulphonic Acid, Para Amino Benzoic Acid and mixtures thereof.

**16.** The cosmetic foundation composition of claim 14 or 15, wherein the water dispersible organic sunscreen is methylene bis-benzotriazolyl tetramethylbutylphenol.

**17.** The cosmetic foundation composition of any one of claims 10 to 16, additionally comprising from 1 to 20%, preferably greater than 2% and less than or equal to 15% by weight of the cosmetic foundation composition of lipophilic organic sunscreen.

**18.** The cosmetic foundation composition of claim 17, wherein the lipophilic organic sunscreen is selected from the group consisting of 2-ethylhexyl-2-cyano-3, 3-diphenylacrylate, 2-ethylhexyl-p-methoxycinnamate, octyl-p-methoxycinnamate, homosalate and mixtures thereof.

**19.** Use of a cosmetic foundation composition of any one of the preceding claims to increase skin translucency and/or as a sunscreen.

**20.** A cosmetic foundation composition having an SPF-value of greater than or equal to 15, preferably greater than or equal to 18, more preferably greater than or equal to 20 and a $\Delta L_T$ value greater than or equal to 0.5, preferably greater than or equal to 1.0, more preferably greater than or equal to 2.5, the composition comprising comprising:

(A) from 0.01% to 15% by weight of the cosmetic foundation composition of cross-linked organopolysiloxane elastomer;
(B) a dispersant;
(C) a sunscreen comprising one or more of C1, C2 and C3:

(C1) first metal oxide sunscreen particles having a first number weighted average primary particle size from 1nm to 50nm and second metal oxide sunscreen particles having a second number weighted average primary particle size which is greater than 50nm and less than or equal to 200nm;
(C2) a water soluble and/or a water dispersible organic sunscreen;
(C3) a lipophilic organic sunscreen;

where $\Delta L_T$ is measured according to the following equation:

$$\Delta L_T = \Delta L_2 - \Delta L_1$$

where:

$$\Delta L_1 = L_1^{110} - L_1^{15};$$

$$\Delta L_2 = L_2^{110} - L_2^{15}$$

$L_1$ is a measurement of $L^y$ taken on bare skin, to which no product has been applied;

$L_2$ is a measurement of $L^y$ taken on skin, to which product has been applied; and
$L^y$ is the translucency value at a specific angle, y, from specular.

**21.** The cosmetic foundation of claim 20, wherein the dispersant:

(a) is non-volatile; and
(b) has a viscosity less than or equal to 5cm$^2$/s (500 centistokes), preferably less than or equal to 1cm$^2$/s (100 centistokes), more preferably less than or equal to 0.5 cm$^2$/s (50 centistokes) at 25°C; and
(c) has a dielectric constant from 3.0 to 5.0, preferably from 3.5 to 5.0.

**22.** The cosmetic foundation of claim 20, wherein the dispersant adheres to the formula R — X — R', wherein R and R' are $C_6$-$C_{10}$ alkyl groups and X is a carbonate group.

**23.** The cosmetic foundation of claim 22, wherein both R and R' are 2-ethylhexyl groups.

**24.** The cosmetic foundation of claim 20, wherein the dispersant is isononyl isononanoate.

**25.** The cosmetic foundation of any one of claims 20 to 24, comprising from 0.1 %wt to 20%wt, preferably from 0.5%wt to 10%wt, more preferably from 1%wt to 8%wt , more preferably still from 2%wt to 6%wt dispersant.

**26.** The cosmetic foundation composition of any one of claims 20 to 25, wherein the water soluble organic sunscreen is selected from the group consisting of 2-Phenylbenzimidazole-5-sulfonic acid, 2-Benzoyl-5-Methoxy-1-Phenol-4-Sulphonic Acid, Para Amino Benzoic Acid and mixtures thereof.

**27.** The cosmetic foundation composition of any one of claims 20 to 26, wherein the water dispersible organic sunscreen is methylene bis-benzotriazolyl tetramethylbutylphenol.

**28.** The cosmetic foundation composition of any one of claims 20 to 27, wherein the lipophilic organic sunscreen is selected from the group consisting of 2-ethylhexyl-2-cyano-3, 3-diphenylacrylate, 2-ethylhexyl-p-methoxycinnamate, octyl-p-methoxycinnamate, homosalate and mixtures thereof.

**29.** The cosmetic foundation composition of any one of claims 20 to 28, comprising from 1 to 20%, preferably greater than 2% and less than or equal to 15% by weight of the cosmetic foundation composition of lipophilic organic sunscreen.

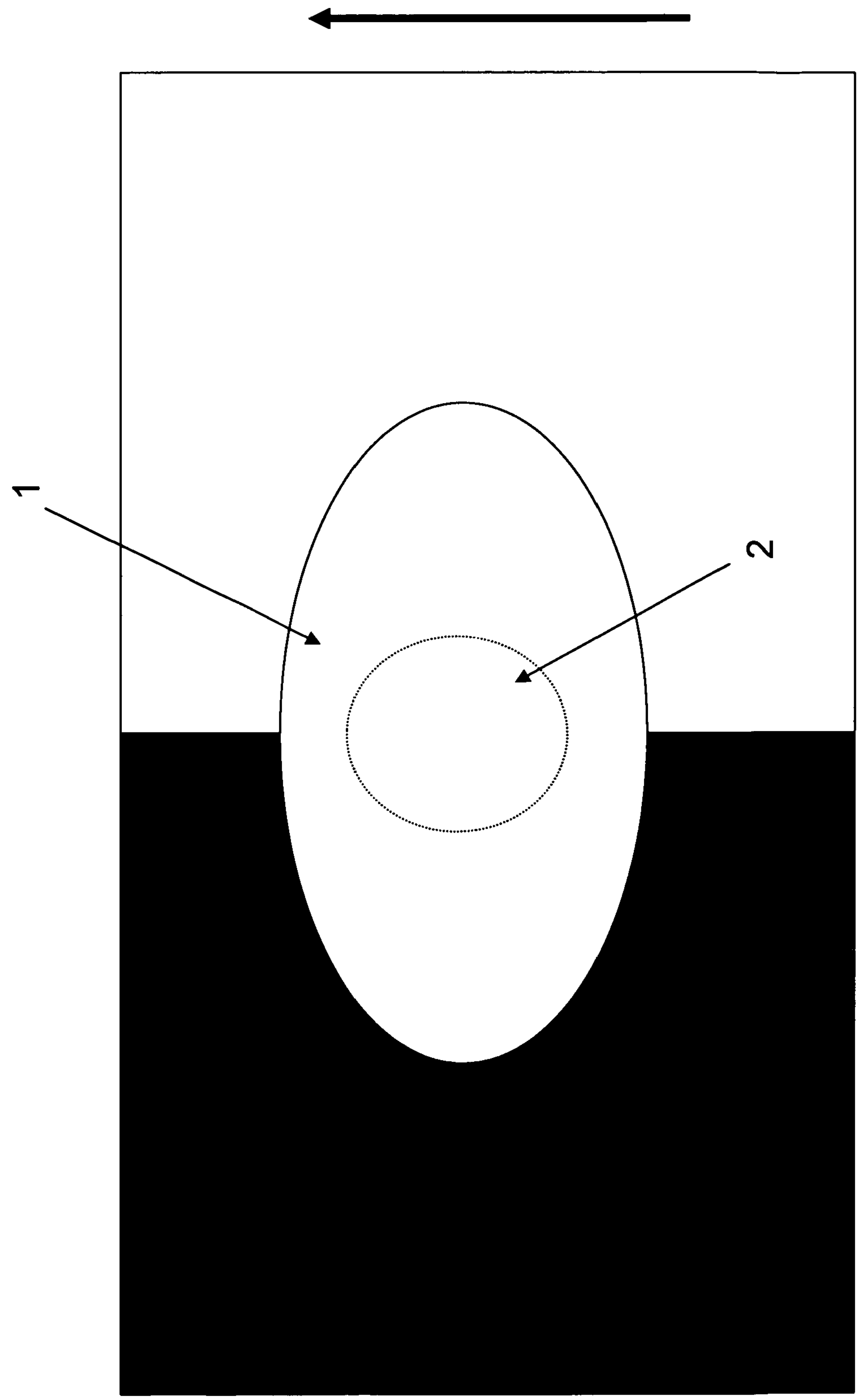


Fig.1

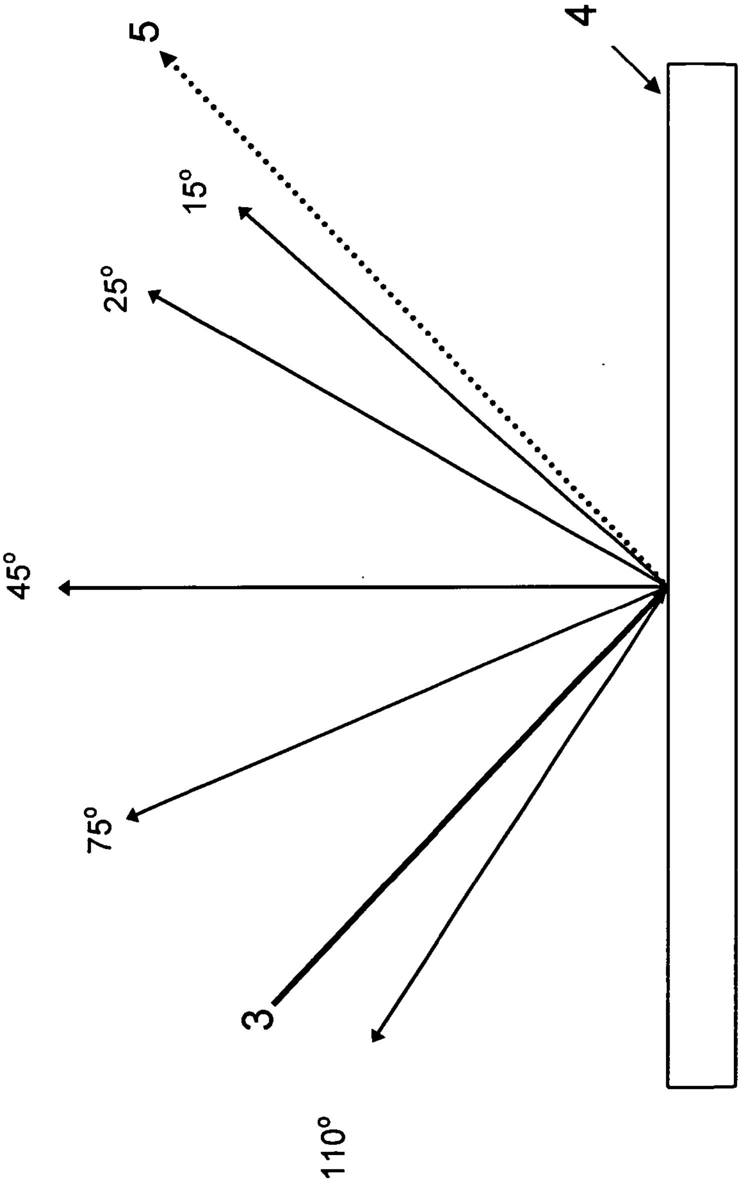


Fig.2

European Patent Office

# PARTIAL EUROPEAN SEARCH REPORT

which under Rule 45 of the European Patent Convention shall be considered, for the purposes of subsequent proceedings, as the European search report

Application Number
EP 05 00 7389

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 2002/028184 A1 (J. SUNKEL ET AL) 7 March 2002 (2002-03-07)<br>* paragraphs [0117], [0155] - [0165]; examples IV,XIII,XIV *<br>----- | 1-5, 8-21, 24-29 | A61K7/021 |
| X | US 2002/028223 A1 (M. VATTER ET AL) 7 March 2002 (2002-03-07)<br>* paragraphs [0155], [0187] - [0198]; claims; examples *<br>----- | 1-5, 8-21, 24-29 | |
| X | US 6 482 441 B1 (HASEGAWA YUKIO ET AL) 19 November 2002 (2002-11-19)<br>* claims; examples 3-1,13-1 *<br>----- | 1,4,5, 8-13, 19-21, 24,25 | |
| X | EP 1 314 415 A (SHIN-ETSU CHEMICAL CO., LTD) 28 May 2003 (2003-05-28)<br>* claim 31; examples 21-23 *<br>-----<br>-/-- | 1-4, 10-20 | TECHNICAL FIELDS SEARCHED (Int.Cl.7)<br>A61K |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

3

| Place of search | Date of completion of the search | Examiner |
|---|---|---|
| Berlin | 16 September 2005 | Boeker, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
&: member of the same patent family, corresponding document

EPO FORM 1503 03.82 (P04C07)

European Patent Office

# PARTIAL EUROPEAN SEARCH REPORT

Application Number
EP 05 00 7389

| Category | Citation of document with indication, where appropriate, of relevant passages | Relevant to claim | CLASSIFICATION OF THE APPLICATION (Int.Cl.7) |
|---|---|---|---|
| X | DATABASE CAPLUS [Online] CHEMICAL ABSTRACTS SERVICE, COLUMBUS, OHIO, US; ITANI, MAMORU ET AL VATTER, MICHAEL LEE ET AL BRUENING, STEFAN ET AL ELLIOTT, DAVID L. ET AL KAWASHIMA, FUMIKO ET AL AVENDANO, EST: "Manufacture of cosmetic compositions containing stably dispersed inorganic particles, and their use for sunscreens" XP002302077 retrieved from STN Database accession no. 2001:159411 * abstract * & JP 2001 058935 A2 (DAINIPPON KASEI CO., LTD., JAPAN; MIKUNI COLOR WORKS CO., LTD. THE PRO) 6 March 2001 (2001-03-06) ----- | 1-4, 10-21 | |
| X | US 6 258 345 B1 (ROUQUET VIOLAINE ET AL) 10 July 2001 (2001-07-10) * examples * ----- | 1-4, 10-20 | TECHNICAL FIELDS SEARCHED (Int.Cl.7) |
| X | EP 1 213 006 A (KANEBO ET AL.) 12 June 2002 (2002-06-12) * paragraphs [0057], [0061] - [0066], [0078]; examples 1,6 * ----- | 1-3,8,9, 11-21, 24,25 | |
| X | EP 1 352 625 A (KAO CORPORATION) 15 October 2003 (2003-10-15) * tables 4-6 * ----- | 1-4, 10-21 | |
| X | EP 1 123 697 A (SHISEIDO CO., LTD) 16 August 2001 (2001-08-16) * tables 3,13 * ----- | 1-4, 10-21 | |
| X | US 2004/091440 A1 (KAMEI MASANAO ET AL) 13 May 2004 (2004-05-13) * claims; examples * ----- | 1-4, 10-21 | |

-/--

3

EPO FORM 1503 03.82 (P04C10)

European Patent Office

# PARTIAL EUROPEAN SEARCH REPORT

Application Number
EP 05 00 7389

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| X | DATABASE CAPLUS [Online]<br>CHEMICAL ABSTRACTS SERVICE, COLUMBUS, OHIO, US;<br>SATO, YUMIKO ET AL: "Transparent or translucent emulsified cosmetic stock"<br>XP002302813<br>retrieved from STN<br>Database accession no. 1998:293269<br>*IT* and<br>* abstract *<br>& JP 10 120524 A2 (KAO CORP., JAPAN)<br>12 May 1998 (1998-05-12)<br>----- | 1 | |
| | | | TECHNICAL FIELDS SEARCHED (Int.Cl.7) |

3

EPO FORM 1503 03.82 (P04C10)

European Patent Office

INCOMPLETE SEARCH
SHEET C

Application Number
EP 05 00 7389

Claim(s) searched incompletely:
1-29

Claim(s) not searched:
-

Reason for the limitation of the search:

Present independent claims 1 and 20 relate to cosmetic foundations defined by reference to two desirable characteristics or properties, namely their "translucency" and defined by the use of parameters defining this "translucency" and an SPF factor. The claims cover all possible products having such a property, whereas the application provides support within the meaning of Article 84 EPC and disclosure within the meaning of Article 83 EPC for only a very limited number of such products. In the present case, the claims so lack support, and the application so lacks disclosure, that a meaningful search over the whole of the claimed scope is impossible.
Independent of the above reasoning, the claims also lack clarity (Article 84 EPC). First, an attempt is made to define the product by reference to a result to be achieved, second, the use of these parameters in the present context is considered to lead to a lack of clarity within the meaning of Article 84 EPC.
It is impossible to compare the parameter "translucency" the applicant has chosen to employ with what is set out in the prior art. It appears that there exists no universally accepted definition for this parameter in the art let alone a measurement method.

The parameter of a difference of statistical averages of differences in luminosity resulting after the product claimed has been applied to skns of a statistically representative sample of caucasian females aged 18 - 35 years in certain dosages and as observed under certain angles while illuminated under a certain angle is not recognisable as technical feature which might distinguish the product claimed from related products disclosed in the prior art.

The parameter aiming at characterising the feature "translucency" merely constitutes a desideratum. The skilled person, being faced with the application, does not find sufficient guidance how to achieve products according to claims 1 or 20 over the entire scope of these claims (Art. 83 EPC).

Claims 1 and 20 furthermore do not meet the requirements of Article 84 EPC since it has to be doubted whether the parameter is reliably reproducible. This is especiallyly true because the parameter in question does define the product claimed in indirect terms only: not the product itself, but a result of the application of the product to skin is described.

Moreover, it is not at all clear why such a complicated construct should be an essential feature of the present invention (Art. 56 EPC).

The lack of clarity is such as to render a meaningful complete search impossible.

European Patent Office

**INCOMPLETE SEARCH SHEET C**

Application Number
EP 05 00 7389

Consequently, the search has been restricted to cosmetic compositions (e.g. foundations) comprising cross-linked organopolysiloxane elastomers and/or metal oxides.

**ANNEX TO THE EUROPEAN SEARCH REPORT ON EUROPEAN PATENT APPLICATION NO.** EP 05 00 7389

This annex lists the patent family members relating to the patent documents cited in the above-mentioned European search report.
The members are as contained in the European Patent Office EDP file on
The European Patent Office is in no way liable for these particulars which are merely given for the purpose of information.

16-09-2005

| Patent document cited in search report | Publication date | Patent family member(s) | Publication date |
|---|---|---|---|
| US 2002028184 A1 | 07-03-2002 | AU 7192801 A | 21-01-2002 |
| | | CA 2412961 A1 | 17-01-2002 |
| | | CN 1533267 A | 29-09-2004 |
| | | CZ 20030084 A3 | 14-05-2003 |
| | | EP 1355624 A2 | 29-10-2003 |
| | | JP 2004502715 T | 29-01-2004 |
| | | MX PA03000262 A | 26-01-2004 |
| | | WO 0203951 A2 | 17-01-2002 |
| US 2002028223 A1 | 07-03-2002 | AU 7193101 A | 21-01-2002 |
| | | WO 0203935 A2 | 17-01-2002 |
| US 6482441 B1 | 19-11-2002 | FR 2795949 A1 | 12-01-2001 |
| | | JP 2001072527 A | 21-03-2001 |
| EP 1314415 A | 28-05-2003 | WO 0203928 A1 | 17-01-2002 |
| | | JP 2002029918 A | 29-01-2002 |
| | | US 2003082218 A1 | 01-05-2003 |
| JP 2001058935 A2 | -- | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |

EPO FORM P0459

For more details about this annex : see Official Journal of the European Patent Office, No. 12/82

**ANNEX TO THE EUROPEAN SEARCH REPORT ON EUROPEAN PATENT APPLICATION NO.** EP 05 00 7389

This annex lists the patent family members relating to the patent documents cited in the above-mentioned European search report.
The members are as contained in the European Patent Office EDP file on
The European Patent Office is in no way liable for these particulars which are merely given for the purpose of information.

16-09-2005

| Patent document cited in search report | Publication date | Patent family member(s) | Publication date |
|---|---|---|---|
| JP 2001058935 A2 | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| | | -- | |
| US 6258345 B1 | 10-07-2001 | BR 9804138 A | 14-12-1999 |
| | | CA 2245989 A1 | 01-04-1999 |
| | | DE 69821180 D1 | 26-02-2004 |
| | | DE 69821180 T2 | 25-11-2004 |
| | | EP 0908175 A1 | 14-04-1999 |
| | | ES 2215282 T3 | 01-10-2004 |
| | | FR 2768926 A1 | 02-04-1999 |
| | | JP 11158030 A | 15-06-1999 |
| | | PL 328909 A1 | 12-04-1999 |
| EP 1213006 A | 12-06-2002 | AU 6734700 A | 26-03-2001 |
| | | CN 1382033 A | 27-11-2002 |
| | | WO 0115658 A1 | 08-03-2001 |
| | | JP 3658561 B2 | 08-06-2005 |
| EP 1352625 A | 15-10-2003 | CN 1449736 A | 22-10-2003 |
| | | US 2004001869 A1 | 01-01-2004 |
| EP 1123697 A | 16-08-2001 | AU 775971 B2 | 19-08-2004 |
| | | AU 6596100 A | 19-03-2001 |
| | | CN 1320031 A | 31-10-2001 |
| | | WO 0113874 A1 | 01-03-2001 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 05 00 7389

This annex lists the patent family members relating to the patent documents cited in the above-mentioned European search report.
The members are as contained in the European Patent Office EDP file on
The European Patent Office is in no way liable for these particulars which are merely given for the purpose of information.

16-09-2005

| Patent document cited in search report | Publication date | Patent family member(s) | Publication date |
|---|---|---|---|
| EP 1123697 A | | US 6749838 B1 | 15-06-2004 |
| US 2004091440 A1 | 13-05-2004 | EP 1424373 A2<br>JP 2004155978 A | 02-06-2004<br>03-06-2004 |
| JP 10120524 A2 | -- | --<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>--<br>-- | |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 05 00 7389

This annex lists the patent family members relating to the patent documents cited in the above-mentioned European search report.
The members are as contained in the European Patent Office EDP file on
The European Patent Office is in no way liable for these particulars which are merely given for the purpose of information.

16-09-2005

| Patent document cited in search report | Publication date | Patent family member(s) | Publication date |
|---|---|---|---|
| JP 10120524 A2 | | --<br>--<br>--<br>--<br>--<br>--<br>--<br>--<br>-- | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4268499 A


### Non-patent literature cited in the description


- *Federal Register,* 21 May 1999, vol. 64 (98), 27666-27693
- CTFA International Cosmetic Ingredient Dictionary and Handbook. The Cosmetic, Toiletry, and Fragrance Association, Inc, 1997, vol. 2, 1672
- **MCCUTCHEON'S.** Detergents and Emulsifiers. 317-324